# EUROPEAN PATENT APPLICATION

(11) **EP 3 703 011 A1**
(43) Date of publication of application: **02.09.2020**
(21) Application number: 19159333.4
(22) Date of filing: 26.02.2019
(51) Int. Cl.: G06T 7/73, G06T 7/13

(54) **INTERVENTIONAL DEVICE TRACKING**

(71) Applicant: Koninklijke Philips N.V., 5656 AG Eindhoven (NL)
(72) Inventor: TERMEER, Maurice Alain, 5656 AE Eindhoven (NL); STEENBAKKERS, Mark Henricus Antonius, 5656 AE Eindhoven (NL); HOPPENBROUWERS, Jurgen Jean Louis, 5656 AE Eindhoven (NL)
(74) Representative: de Haan, Poul Erik

(57) **Abstract**

The present invention relates to interventional device tracking. In order to provide a facilitated tracking of instruments with less X-ray radiation, a system (10) for tracking an interventional device is provided that comprises an interface unit (12), a data storage unit (14), a processing unit (16) and an output unit (18). The interface unit is configured to provide at least two current 2D images of a region of interest of a subject acquired with optical cameras in at least two different viewing directions. The 2D images show an instrument in the region of interest, wherein the instrument has a geometry that comprises a rigid cylinder. The processing unit is configured to identify at least one straight line, representing the instrument, in each of the at least two current 2D images. The processing unit is further configured to determine an instrument 3D line based on the identified at least one straight line in each of the at least two current 2D images. The processing unit is also configured to project the instrument 3D line on pre-interventional 3D data of the region of interest of the subject generating a combined navigation image. The output unit is configured to provide the combined navigation image to the user.

## Description

### FIELD OF THE INVENTION

The present invention relates to interventional device tracking, and relates in particular to a system for tracking an interventional device, to a medical imaging arrangement with interventional device tracking for navigation purposes and to a method for tracking of an instrument.

### BACKGROUND OF THE INVENTION

Optical tracking systems for use as a navigation system during surgery are increasingly used in the medical industry. For example, a navigation system provides real-time information on the location of a surgical instrument relative to the patient. This may be useful, for example, during minimal invasive image guided interventions, which are increasingly used alternatively to open surgery procedures. Navigation systems may use multiple infrared cameras combined with special markers that need to be attached to the elements that need to be tracked by the system. Attaching tracked markers to the end of the instrument outside the patient thus allows a tracking system to compute the location of the end of the instrument inside the patient. While the use of infrared markers results in reliable and accurate tracking, attaching these markers to surgical instruments may be considered cumbersome, e.g. undesirable as it inhibits freedom of movement and adds additional mass to the instrument. Instrument tracking is also possible using visible-light cameras and markers, which shares the same downsides as the infrared tracking technologies. Alternatively, a small pattern can be attached to the shaft of the instrument. This pattern is designed such that it can be easily detected by the optical cameras, resulting in similar tracking performance. While this approach solves the mechanical disadvantages of attaching movement-inhibiting markers, it requires specially produced instruments. WO 2013 190409 A2 relates to tracking an interventional device. In order to provide a facilitated and simplified real-time tracking of an interventional device, a medical imaging system for tracking an interventional device is provided, that comprises an interface unit providing first image data of a first part of an interventional device, which first part is arranged outside an object. The first image data comprises 3D image data, e.g. acquired by visible light cameras. The interface unit also provides second image data of a second part of the interventional device, which second part is a continuation of the first part, and which second part is arranged inside the object. The second image data comprises 2D image data, e.g. acquired by X-ray imaging. A processing unit computes a first 3D model portion of the interventional device based on the first image data, and a second 3D model portion of the interventional device based on the second image data and the first model portion. The interface unit provides data of a graphical representation of the interventional device based on the first and second 3D model portions. However, it has been shown that there is an increasing demand for further X-ray dose reduction.

### SUMMARY OF THE INVENTION

There may thus be a need to provide an even more facilitated tracking of instruments that uses less X-ray radiation.

The object of the present invention is solved by the subject-matter of the independent claims; further embodiments are incorporated in the dependent claims. It should be noted that the following described aspects of the invention apply also for the system for tracking an interventional device, for the medical imaging arrangement with interventional device tracking for navigation purposes and for the method for tracking of an instrument.

According to the present invention, a system for tracking an interventional device is provided. The system comprises an interface unit, a data storage unit, a processing unit and an output unit. The interface unit is configured to provide at least two current 2D images of a region of interest of a subject acquired with optical cameras in at least two different viewing directions. The 2D images show an instrument in the region of interest, wherein the instrument has a geometry that comprises a rigid cylinder. The processing unit is configured to identify at least one straight line, representing the instrument, in each of the at least two current 2D images. The processing unit is also configured to determine, i.e. to generate by calculation, an instrument 3D line based on the identified at least one straight line in each of the at least two current 2D images. The processing unit is also configured to project the instrument 3D line on pre-interventional 3D data of the region of interest of the subject generating a combined navigation image. The output unit is configured to provide the combined navigation image to the user.

This provides device tracking, in particular live tracking, based on optical imaging without the need to acquire further X-ray images. The image data for the interior, i.e. the non-visible part of the object, is provided by X-ray image data that is already available and does hence not require an extra, avoidable radiation dose.

In an example, the instrument is an interventional device like a needle, screwdriver, drill or the like.

According to an example, for the identifying of the at least one straight line, the processing unit is configured to provide edge detection filtering of each of the at least two current 2D images. The processing unit is configured to transform the edge detection filtered 2D images, wherein points on a line in 2D image space are transformable to lines in parameter space. Further, peaks in the parameter space are identifiable to determine location of the at least one line in the respective 2D image space.

The edge detection provides improved image data for the identification and location process of the 2D lines (in the image) and thus the instrument to achieve 3D data for these.

According to an example, for the edge detection filtering of each of the at least two current 2D images, the processing unit is configured to apply at least one of a Canny edge detector and a Laplace-filter. For the transforming of the edge detection filtered 2D images, the processing unit is configured to apply at least one of the group of a Hough transformation and a Radon transformation.

This provides robust and reliable identification of the lines and determination of their spatial arrangement.

According to an example, the processing unit is configured to spatially register the 3D scan and the current 2D images in relation to each other. For the identifying of the at least one straight line, the processing unit is configured to use a planned instrument path in a pre-interventional 3D scan to define the region of interest in the at least two current 2D images.

This limits the search space for the image processing chain.

According to an example, the processing unit is configured to identify at least one pair of straight lines representing both sides of the instrument in each of the at least two current 2D images. For the identification of the at least one pair of straight lines, the processing unit is configured to use a width of the instrument to select pairs of straight lines with a distance to each other matching the width.

This further improves accuracy of the identification and facilitates the findings and thus reduces the computation effort.

According to an example, the output unit comprises a display configured to display combined navigation image.

The information in form of the combined navigation image is thus provided to the user, for example as live information.

According to the present invention, also a medical imaging arrangement with interventional device tracking for navigation purposes is provided. The arrangement comprises an imaging system comprising at least two visible-light cameras, and a system for tracking an interventional device according to one of the preceding examples. The imaging system is configured to acquire and to provide the least two current 2D images of the region of interest of a subject to the interface unit.

According to an example, it is further provided a medical X-ray imaging system comprising an X-ray source and an X-ray detector. The medical X-ray imaging system is configured to provide the image data for reconstructing the pre-interventional 3D data.

According to the present invention, also a method for tracking of a medical instrument is provided. The method comprises the following steps:
a) providing at least two current 2D images of a region of interest of a subject acquired with optical cameras in at least two different viewing directions; wherein the 2D images show an instrument in the region of interest; and wherein the instrument has a geometry that comprises a rigid cylinder;
b) identifying at least one straight line, representing the instrument, in each of the at least two current 2D images;
c) determining an instrument 3D line based on the identified at least one straight line in each of the at least two current 2D images;
d) projecting the instrument 3D line on pre-interventional 3D data of the region of interest of the subject generating a combined navigation image; and
e) providing the combined navigation image to the user.

As an example, the determining of the 3D line in step c) is based on epipolar geometry and the known spatial relation of the at least two different viewing directions.

According to an aspect, markers are not required to be attached to the instrument. Also, any other modifications to the instrument are not required. Hence, any existing surgical instrument can be tracked, provided its geometry is primarily a rigid cylinder of fixed diameter, e.g. needles, screwdrivers, drills or the like. An edge detection filter, like the Canny edge detector, is used on the region of interest. In an example, a localized Hough transform is used to detect straight lines in the edge detector response image. The planned path provides the expected position and orientation of the instrument, so only a small range of angles and translations needs to be considered in the Hough transform. The left and right sides of the instrument will form peaks in the image, but any specular reflection in the center of the instrument may do so, too. In an example, the width of the instrument is used to estimate the expected distance between the lines in the camera image and to select the correct pair of peaks in the Hough transform image. In summary, domain knowledge is used to greatly limit the search space and eliminate false positives.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic illustration of a system for tracking an interventional device.
Fig. 2 shows a schematic illustration of a medical imaging arrangement with interventional device tracking for navigation purposes.
Fig. 3 shows an example of a medical imaging arrangement with a medical X-ray imaging system.
Fig. 4 shows basic steps of an example of a method for tracking of an instrument.
Fig. 5A shows an example of an image from a visible-light camera.
Fig. 5B shows an example of an intermediate edge detection step of the image of Fig. 5A.
Fig. 5C shows an example of a Hough transform of the edge detection image of Fig. 5B.
Fig. 6 shows examples of three different views with a projected 3D line of the instrument.
Fig. 7A shows a first example of a detected instrument line projected onto a 3D scan image.
Fig. 7B shows a second example of a detected instrument line projected onto a 3D scan image.
Fig. 8 shows an example of a camera view that is used to position the instrument inside the region of interest in which the instrument can be tracked.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

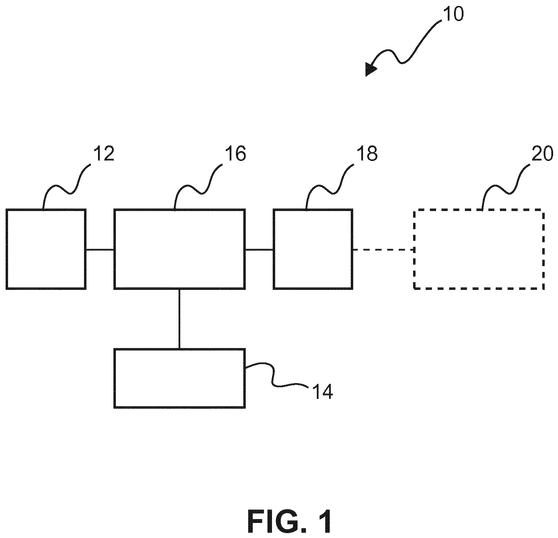

Fig. 1 shows a schematic illustration of a system 10 for tracking an interventional device. The system 10 comprises an interface unit 12, a data storage unit 14, a processing unit 16 an output unit 18. The interface unit 12 is configured to provide at least two current 2D images of a region of interest of a subject acquired with optical cameras in at least two different viewing directions. The 2D images show an instrument in the region of interest, wherein the instrument has a geometry that comprises a rigid cylinder. The processing unit 16 is configured to identify at least one straight line, representing the instrument, in each of the at least two current 2D images. The processing unit 16 is also configured to determine an instrument 3D line based on the identified at least one straight line in each of the at least two current 2D images. The processing unit 16 is further configured to project the instrument 3D line on pre-interventional 3D data of the region of interest of the subject generating a combined navigation image. The output unit 18 is configured to provide the combined navigation image to the user.

The term "unit" relates to the particular function. The units can be provided as separate parts or as an integrated component. The interface unit can also be referred to as interface; the data storage unit can also be referred to as data storage; the processing unit can also be referred to as processor; and the output unit can also be referred to as output.

The instrument is provided as a straight instrument, e.g. a needle or the like.

The tracking of the instrument is thus provided as a training-less procedure, i.e. no learning or the like for the detection of the instrument in the images is required.

Markers like skin markers on the patient can be provided for tracking and thus registering the subject. The patient can also be registered via anatomical or artificial landmarks.

In an example, the at least two current 2D images are provided by visible-light cameras. The pre-interventional 3D data is provided by X-ray imaging.

In an example, the pre-interventional 3D data is based on cone-beam CT imaging.

In an example, the surgical instruments that are tracked are straight and have a known length (or the length is calibrated before use) and a known diameter.

Once the 3D line corresponding to the instrument location is known, this line can be projected on the camera images as well as on the previously acquired 3D scan. The latter allows the surgeon to navigate on the anatomy of the patient e.g. without the use of fluoroscopy.

The term "current 2D images" relates to 2D images of a current situation, e.g. live images or near live. The current images are also referred to as live images.

The term "at least two different viewing directions" relates to images taken in different orientation to each other such that the spatial orientation provides additional 3D information in addition to the 2D information of the respective images.

The term "rigid cylinder" relates to a stiff part of the device provided for insertion into e.g. a subject. "Rigid" relates to the capability of insertion with only small degree of deformation.

The term "straight line, representing the instrument" relates to an essentially linear line in the image.

The term "instrument 3D line" relates to a line in the spatial domain, i.e. in 3D, which line represents the visible part of the instrument.

The term "project the instrument 3D line" relates to generating a projection of the 3D line under a given viewing direction.

The term "pre-interventional 3D data of the region of interest of the subject" relates to e.g. 3D image data or 3D model data that is based on data or image acquisition performed beforehand, e.g. by ultrasound imaging or X-ray imaging

The term "generating" relates to creating the image showing more information than the 2D image itself.

The term "combined navigation image" relates to an enhanced 2D image that provides additional information to the user, which is supporting a navigating task of the instrument.

In an example, the region of interest used by the line detection algorithm is smaller than the region of interest imaged by the cameras. For example, a physician indicates a desired screw location on the cone-beam CT scan. The centerline of the screw is extended throughout the scan and the intersection between the patient's skin and this line is computed. A region of interest is defined as a cylinder with a central axis between this point and a few centimeters (e.g. between approximately 5 cm and 15 cm, e.g. approximately 8 cm) further along the line and a diameter of a few centimeters (e.g. between approximately 1 cm and 5 cm, e.g. approximately 3 cm). The region of interest could be another shape, e.g. a cone could also be a choice. This region is smaller than the volume imaged by the cameras, which may contain e.g. half the patient.

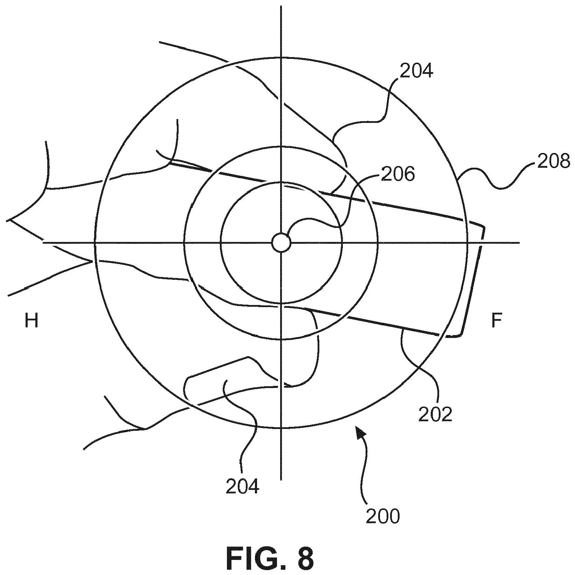

The instrument is only tracked within the region of interest of the line detection algorithm. For positioning the instrument within this region of interest, the centerline of this region can be projected onto the camera images. This is particularly helpful if one of the cameras is positioned such that this centerline is exactly perpendicular to the camera image (see Fig. 8). If the cameras are attached to a motorized positioning system, e.g. a cone-beam CT scanner, such a camera position can be automatically computed and one of the 2D cameras can be automatically positioned to assist with the initial positioning of the instrument.

In an option, for the identifying of the at least one straight line, the processing unit 16 is configured to provide edge detection filtering of each of the at least two current 2D images. The processing unit 16 is configured to transform the edge detection filtered 2D images, wherein points on a line in 2D image space are transformable to lines in parameter space. Peaks in the parameter space are identifiable to determine location of the at least one line in the respective 2D image space.

In a further option, for the edge detection filtering of each of the at least two current 2D images, the processing unit 16 is configured to apply at least one of a Canny edge detector and a Laplace-filter. Further, for the transforming of the edge detection filtered 2D images, the processing unit 16 is configured to apply at least one of the group of a Hough transformation and a Radon transformation.

In an option, the processing unit 16 is configured to complement the 3D line, the3D line representing a visible part of the instrument, by an augmented 3D section of the instrument, the augmented 3D section representing a non-visible part of the instrument that is partly inserted into the subject.

In an option, the processing unit 16 is configured to spatially register the 3D scan and the current 2D images in relation to each other. For the identifying of the at least one straight line, the processing unit 16 is configured to use a planned instrument path in a pre-interventional 3D scan to define the region of interest in the at least two current 2D images.

In an option, the processing unit 16 is configured to identify at least one pair of straight lines representing both sides of the instrument in each of the at least two current 2D images. For the identification of the at least one pair of straight lines, the processing unit 16 is configured to use a width of the instrument to select pairs of straight lines with a distance to each other matching the width.

In an example, two lines in each image are detected, the two lines corresponding to the sides of the projected instrument. The average of these two lines provides the (projected) centerline, which, in an example, is subsequently used for triangulation.

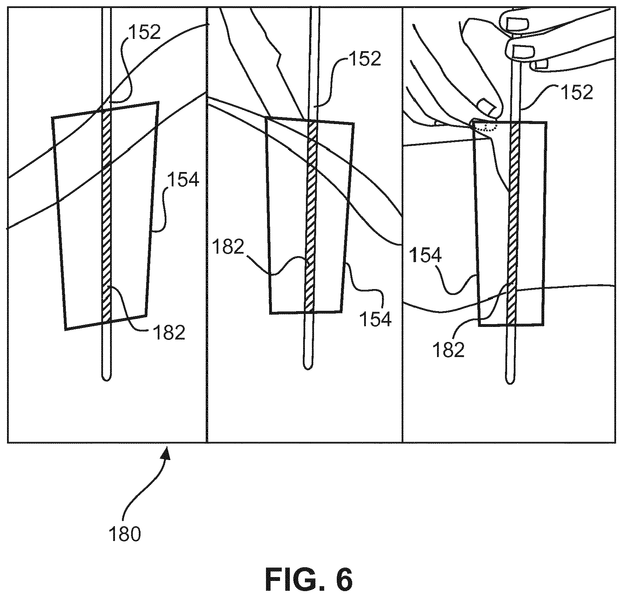

In an option, the processing unit 16 is configured to project the instrument 3D line onto at least one of the 2D images to provide a verification image. The verification image is then provided to the user to verify the correct detection of the instrument (see also Fig. 6).

In an option, as indicated with hashed lines, the output unit 18 comprises a display 20 configured to display the combined navigation image.

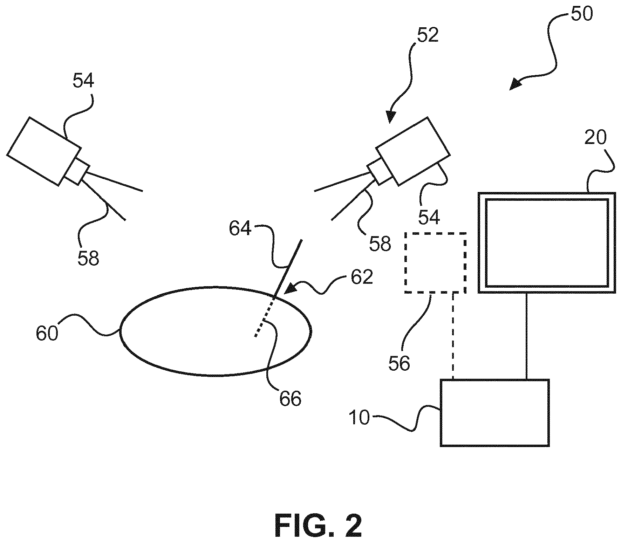

Fig. 2 shows a schematic illustration of a medical imaging arrangement 50 with interventional device tracking for navigation purposes. The arrangement comprises an imaging system 52 comprising at least two visible-light cameras 54 and an example of the system 10 for tracking an interventional device according to one of the preceding examples. The imaging system 52 is configured to acquire and to provide the least two current 2D images of the region of interest of a subject to the interface unit 12.

The visible-light cameras 54 are provided, in an example, as video cameras.

In an option, as indicated with hashed lines, a registration unit 56 is provided configured to register the at least two current 2D images with the pre-interventional 3D data of the region of interest of the subject.

In Fig. 2, image capturing by the cameras is indicated with lines 58. Further, an object 60 is illustrated. An instrument 62 is partly inserted, as indicated with a first part 64 outside the object 60, and a second part 66 in hashed lines being inserted.

In an example, during an acquisition of image data for generating the pre-interventional 3D data, the subject is registered with the image acquisition system, e.g. an X-ray imaging system. Further, the visible-light cameras are rigidly attached to the image acquisition system, i.e. the spatial arrangement of the cameras in relation to the X-ray imaging system are known. Hence, the cameras are registered with the X-ray imaging system. Still further, the at least two current 2D images are registered with the image acquisition system. Thus, the pre-interventional 3D data and the at least two current 2D images are spatially registered to each other.

In an example, the visible-light cameras are rigidly linked to the X-ray source and the X-ray detector of the medical X-ray imaging system. The subject is tracked in relation to this device world during the image acquisition of i) the image data for reconstructing the pre-interventional 3D data and ii) the at least two current 2D images.

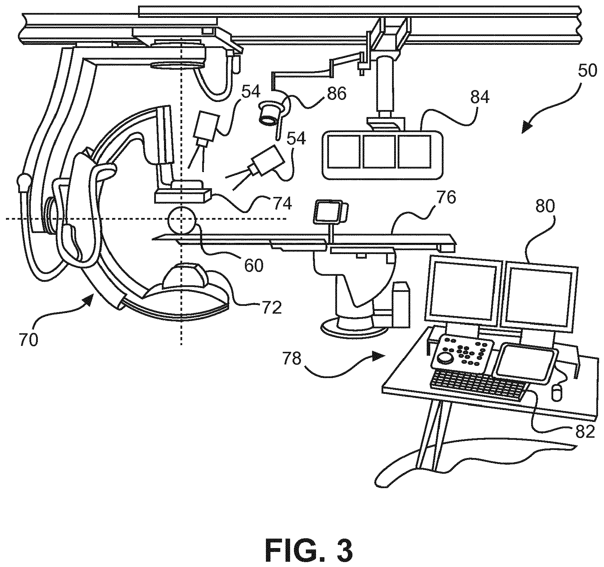

Fig. 3 shows an example of the medical imaging arrangement with a medical X-ray imaging 70 comprising an X-ray source 72 and an X-ray detector 74. The medical X-ray imaging system 70 is configured to provide the image data for reconstructing the pre-interventional 3D data. The object 60 is shown on a patient support 76, and a console 78 for control, with a display arrangement 80 and an interface arrangement 82, is shown in the foreground. Further, additional displays 84 and lighting equipment 86 are provided as options.

The cameras 54 allow tracking of an instrument (not shown in Fig. 3) without the active use of X-ray. The X-ray imaging system may be used for pre- or intra-operational imaging in order to achieve the 3d data.

In an example, not further shown in detail, an augmented reality surgical navigation system is provided that comprises an interventional X-ray system with four high-resolution optical cameras attached rigidly to the X-ray detector. The location of the optical cameras relative to each other and to the X-ray detector is calibrated. At the start of the procedure, a 3D X-ray scan is acquired of the anatomy of interest. The surgeon plans the intended path of the instrument on the 3D scan.

In another example, more than two cameras are provided leading to multiple 3D lines. The processing unit 16 is configured to back-project each 3D line to each camera image. The processing unit 16 is configured to select the 3D line with the lowest re-projection error as the instrument 3D line for the combined navigation image.

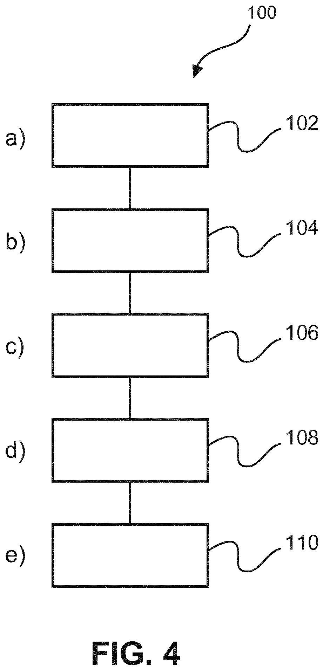

Fig. 4 shows basic steps of an example of a method 100 for tracking of an instrument. The method comprises the following steps:
- In a first step 102, also referred to as step a), at least two current 2D images of a region of interest of a subject are provided that are acquired with optical cameras in at least two different viewing directions. The 2D images show an instrument in the region of interest, and the instrument has a geometry that comprises a rigid cylinder.
- In a second step 104, also referred to as step b), at least one straight line is identified, representing the instrument, in each of the at least two current 2D images.
- In a third step 106, also referred to as step c), an instrument 3D line is determined based on the identified at least one straight line in each of the at least two current 2D images.
- In a fourth step 108, also referred to as step d), the instrument 3D line is projected on pre-interventional 3D data of the region of interest of the subject generating a combined navigation image.
- In a fifth step 110, also referred to as step e), the combined navigation image is provided to the user.

The navigation is based on 3D pre-interventional image data plus 2D visible-light video imaging as live images. Domain knowledge is used to greatly limit the search space and eliminate false positives.

The rigid cylinder is of a fixed and known diameter.

In an example, an edge detection filter in combination with a localized Hough transform is used to find straight lines in each camera image, corresponding to both sides of the instrument. The width of the instrument is used to select the correct line pair.

In step c), the 2D lines are triangulated to 3D lines.

In an example, pairs of 2D lines are triangulated to 3D lines and the 3D line with the lowest re-projection error is selected as the instrument line.

In an example, in step c), the three-dimensional arrangement of the line is determined, i.e. the spatial relation of the line.

The determining of the 3D line in step c) is based on epipolar geometry and the known spatial relation of the at least two different viewing directions.

In an example, the 3D line is projected on the 3D scan so the surgeon can navigate inside the patient using only visible-light video imaging.

In an example, in step e), the combined navigation image is displayed to the user as live image.

In an example, in step b), at least one pair of straight lines representing both sides of the instrument in each of the at least two current 2D images is identified.

In an example, for the identifying of the at least one straight line in step b), it is provided the sub-steps of:
b1) edge detection filtering of each of the at least two current 2D images; and
b2) transforming the edge detection filtered 2D images, wherein points on a line in 2D image space are transformed to lines in parameter space, and wherein peaks in the parameter space are identified to determine location of the at least one line in the respective 2D image space.

In an example, in step d), the 3D line representing a visible part of the instrument is complemented by an augmented 3D section of the instrument, the augmented 3D section representing a non-visible part of the instrument that is partly inserted into the subject.

The augmented 3D section is also referred to as extended part, or as extended inside part of the instrument, or as extended 3D line, or augmented 3D line or augmented 3D portion.

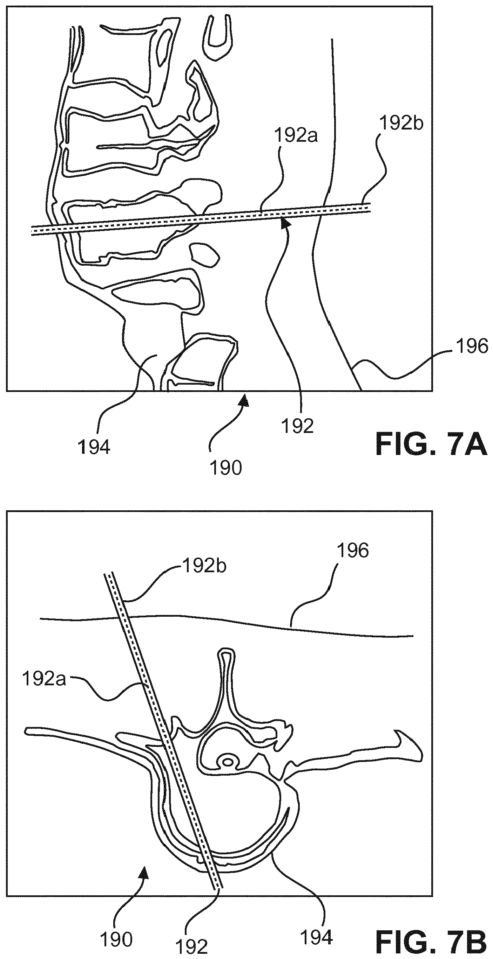

In an example, at least two different projections in two different viewing angles of the combined navigation image pre-interventional 3D are shown (see Fig. 7A and Fig. 7B).

In an example, for step b1) of edge detection filtering of each of the at least two current 2D images, it is provided at least one of the group of a Canny edge detector and a Laplace-filter. Further, for step b2) of transforming the edge detection filtered 2D images, it is provided at least one of the group of a Hough transformation and a Radon transformation.

In an example, for step b), a planned instrument path in a pre-interventional 3D scan is used to define the region of interest in the at least two current 2D images. The 3D scan and the current 2D images are spatially registered in relation to each other.

In an example, the planned instrument path is used as a central axis of a cylinder with a predetermined radius that is subsequently projected on each of the camera images. The projected cylinder serves as a region of interest for the image processing chain. Having a small region of interest greatly reduces the search space and eliminates many false positives. This is of advantage, as processing the full camera images would yield too many candidates and may make it very difficult to select the correct instrument location.

The planned instrument path can also be provided as determined instrument path. The planned instrument path can also be referred to as target path. The planned instrument path can also be derived from tracking a catheter inserted beforehand for examination purposes to detect the optimal path for the instrument.

In an example, in step b), a width of the instrument is used to select pairs of straight lines with a distance to each other matching the width. The width of the instrument is taken for orientation for an easier identification procedure.

In an example, the left and right sides of the instrument will form peaks in the image of the Hough transform and based on the width of the instrument, the expected distance is estimated to select the correct pair of peaks in the Hough transform image.

In an example, the width of the instrument is used to de-select pairs of straight lines with a non-matching distance to each other.

In case of correct detection, the projected instrument 3D line and the instrument visible in the 2D image are aligned to each other, i.e. they are matching.

The term "subject" may also be referred to as individual. The "subject" may further also be referred to as patient, although it is noted that this term does not indicate whether any illness or disease is actually present with the subject.

In the following, some of the above-mentioned stages of the tracking procedure are further illustrated.

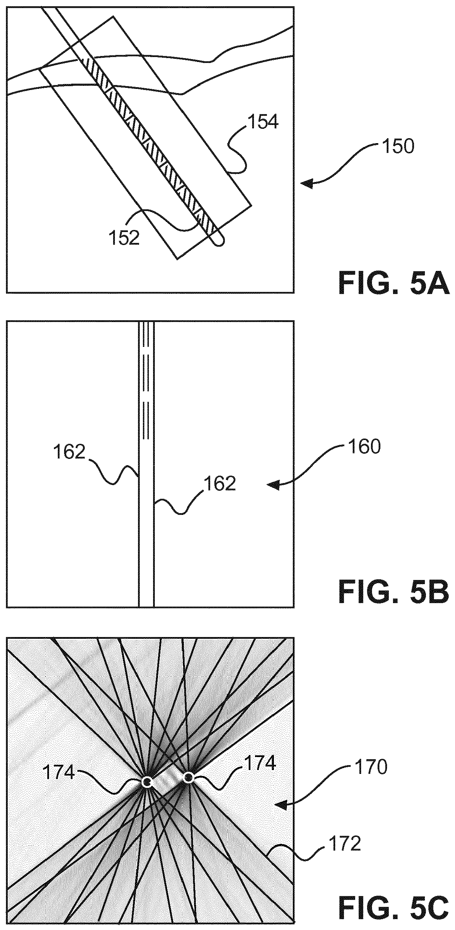

Fig. 5A shows an example of an image 150 from a visible-light camera. An instrument 152 is shown, e.g. a needle partly inserted into an object. Further, a frame 154 indicates a region of interest. This may be derived from a planned instrument path in a pre-interventional 3D scan.

Fig. 5B shows an example of an intermediate edge detection step 160 of the image of Fig. 5A. A pair of lines 162 is shown as a result. The two lines 162 represent the two outlines of the instrument 152 of Fig. 5A. The two lines have ben rotated for better visibility.

Fig. 5C shows an example of a Hough transform 170 of the edge detection image of Fig. 5B. The points along the two lines result in several lines 172 that are crossing each other and form peaks 174. The peaks represent the two boundary lines of the instrument 152.

In an option, the processing unit 16 is configured to project the instrument 3D line onto at least one of the 2D images to provide a verification image; and the output unit 18 is configured to provide the verification image to the user to verify the correct detection of the instrument.

In an example, even if optically tracking the instrument may fail at any time during a procedure, for example if the instrument is occluded by the surgeon's hands, feedback about the result of the instrument detection is provided by providing a viewport with e.g. three camera images that are cropped to the part of the image used for instrument detection and rotated such that the expected instrument direction coincides with the vertical axis of the image. The detected instrument is projected on top of these images. In this viewport, the surgeon can quickly see whether the instrument is visible to the cameras and whether the detection is correct. Fig. 6 shows examples of three different views 180 of the instrument 152 with a projected 3D line 182 of the instrument. The frames 154 are also shown. For better visibility, the visible light images are aligned to show the instrument in a vertical orientation. The user can verify if the 3D lines (for the instrument) are correctly projected matching with the instrument shown in the image.

Fig. 7A shows a first example of a 3D scan image 190 with a detected instrument line 192 projected onto the 3D scan image 190 showing an anatomic structure 194, for example vertebras. A skin surface line 196 indicates the object's outer boundary. Hence, a part 192a of the instrument line 192 that is arranged within the object is reconstructed and supplemented to a part 192b derived from the optical images. Fig. 7A thus shows a combined navigation image.

Fig. 7B shows a second example of a detected instrument line projected onto a 3D scan image. Same reference numerals are used as in Fig. 7A. For example, the second view may be a projection of the same situation as in Fig. 7A, but in a different orientation, e.g. perpendicular to the projection direction of Fig. 7A. Fig. 7B thus also shows a combined navigation image.

Fig. 8 shows an example of a camera image 200 such that the planned path and thus the centerline of the region of interest is perpendicular to the camera image. An instrument 202 is held by a surgeon's fingers 204 such that a part 206 to be inserted is facing towards the camera. Cross-hairs 208 indicate the centerline of the planned path. If the back of the instrument is centered within the cross-hairs, the instrument is approximately aligned with the planned path and it can be tracked by the instrument tracking method.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A system (10) for tracking an interventional device, the system comprising:
- an interface unit (12);
- a data storage unit (14);
- a processing unit (16); and
- an output unit (18);
wherein the interface unit is configured to provide at least two current 2D images of a region of interest of a subject acquired with optical cameras in at least two different viewing directions; wherein the 2D images show an instrument in the region of interest, wherein the instrument has a geometry that comprises a rigid cylinder;
wherein the processing unit is configured to identify at least one straight line, representing the instrument, in each of the at least two current 2D images; to determine an instrument 3D line based on the identified at least one straight line in each of the at least two current 2D images; and to project the instrument 3D line on pre-interventional 3D data of the region of interest of the subject generating a combined navigation image; and
wherein the output unit is configured to provide the combined navigation image to the user.

2. System according to claim 1, wherein, for the identifying of the at least one straight line, the processing unit is configured to provide edge detection filtering of each of the at least two current 2D images; and to transform the edge detection filtered 2D images;
wherein points on a line in 2D image space are transformable to lines in parameter space; and
wherein peaks in the parameter space are identifiable to determine location of the at least one line in the respective 2D image space.

3. System according to claim 2, wherein, for the edge detection filtering of each of the at least two current 2D images, the processing unit is configured to apply at least one of a Canny edge detector and a Laplace-filter; and
wherein, for the transforming of the edge detection filtered 2D images, the processing unit is configured to apply at least one of the group of a Hough transformation and a Radon transformation.

4. System according to claim 1, 2 or 3, wherein the processing unit is configured to complement the 3D line representing a visible part of the instrument by an augmented 3D section of the instrument; and
wherein the augmented 3D section represents a non-visible part of the instrument that is partly inserted into the subject.

5. System according to one of the preceding claims, wherein the processing unit is configured to spatially register the 3D scan and the current 2D images in relation to each other; and
wherein, for the identifying of the at least one straight line, the processing unit is configured to use a planned instrument path in a pre-interventional 3D scan to define the region of interest in the at least two current 2D images.

6. System according to one of the preceding claims, wherein the processing unit is configured to identify at least one pair of straight lines representing both sides of the instrument in each of the at least two current 2D images; and
wherein, for the identification of the at least one pair of straight lines, the processing unit is configured to use a width of the instrument to select pairs of straight lines with a distance to each other matching the width.

7. System according to one of the preceding claims, wherein the processing unit is configured to project the instrument 3D line onto at least one of the 2D images to provide a verification image; and
wherein the output unit is configured to provide the verification image to the user to verify the correct detection of the instrument.

8. System according to one of the preceding claims, wherein the output unit comprises a display configured to display combined navigation image.

9. A medical imaging arrangement (50) with interventional device tracking for navigation purposes, the arrangement comprising:
- an imaging system (52) comprising at least two visible-light cameras (54); and
- a system (10) for tracking an interventional device according to one of the preceding claims;
wherein the imaging system is configured to acquire and to provide the least two current 2D images of the region of interest of a subject to the interface unit.

10. Medical imaging arrangement according to claim 9, wherein a registration unit (56) is provided configured to register the at least two current 2D images with the pre-interventional 3D data of the region of interest of the subject.

11. Medical imaging arrangement according to claim 9 or 10, wherein more than two cameras are provided leading to multiple 3D lines; and
wherein the processing unit is configured to back-project each 3D line to each camera image, and to select the 3D line with the lowest re-projection error as the instrument 3D line for the combined navigation image.

12. Medical imaging arrangement according to claim 9, 10 or 11, wherein it is further provided:
- a medical X-ray imaging (60) system comprising an X-ray source (62) and an X-ray detector (64);
wherein the medical X-ray imaging system is configured to provide the image data for reconstructing the pre-interventional 3D data.

13. A method (100) for tracking of an instrument, comprising the following steps:
a) providing (102) at least two current 2D images of a region of interest of a subject acquired with optical cameras in at least two different viewing directions, wherein the 2D images show an instrument in the region of interest, wherein the instrument has a geometry that comprises a rigid cylinder;
b) identifying (104) at least one straight line, representing the instrument, in each of the at least two current 2D images;
c) determining (106) an instrument 3D line based on the identified at least one straight line in each of the at least two current 2D images;
d) projecting (108) the instrument 3D line on pre-interventional 3D data of the region of interest of the subject generating a combined navigation image; and
e) providing (110) the combined navigation image to the user.

14. A computer program element for controlling an apparatus according to one of the claims 1 to 12, which, when being executed by a processing unit, is adapted to perform the method steps of claim 13.

15. A computer readable medium having stored the program element of claim 14.
